# EUROPEAN PATENT APPLICATION

(11) **EP 3 611 271 A1**
(43) Date of publication of application: **19.02.2020**
(21) Application number: 18798716.9
(22) Date of filing: 08.03.2018
(51) Int. Cl.: C12Q 1/24, C12M 1/26, C12M 3/00, C12Q 1/02, G01N 1/04

(54) **PRETREATMENT METHOD FOR CELL MIGRATION AND CELL MIGRATION DEVICE**

(30) Priority: 09.05.2017 JP 2017093026
(71) Applicant: Yamaha Hatsudoki Kabushiki Kaisha, Iwata-shi, Shizuoka-ken 438-8501 (JP)
(72) Inventor: IZUME, Yohei, Shizuoka-ken 438-8501 (JP); SAKAMOTO, Masaru, Shizuoka-ken 438-8501 (JP)
(74) Representative: Müller-Boré & Partner Patentanwälte PartG mbB
(86) International application number: PCT/JP2018/009104
(87) International publication number: WO 2018/207450

(57) **Abstract**

A pretreatment method for cell transfer is a pretreatment method to be performed for transferring a required cell to a predetermined transfer destination by picking the cell, from a mass containing a plurality of cells of different sizes, using a cell transfer device including cell picking means, the pretreatment method including a classification step of classifying the required cell, from among the plurality of cells contained in the mass, into at least two groups according to a size of the cell; and a supply step of supplying the classified cells, in a state of being individually classified, to a position where picking can be conducted by the picking means.

## Description

### Technical Field

The present invention relates to a pretreatment method to be performed for picking a required cell by the use of a cell transfer device including cell picking means and transferring the cell to a predetermined transfer destination, and a cell transfer device to which the method is applied.

### Background Art

For example, in applications for medical or biological research, there is a case where a single cell, or a cellular aggregate which is a result of three-dimensional agglomeration of cells, or a cell mass obtained by agglomerating and culturing a piece of a cell (hereinafter, simply referred to as a cell in the present specification) is stored in a well of a microplate having wells aligned in a matrix for the purpose of processing work such as observation, checking of efficacy of medicines, examination, or culture. A cell to be stored in the well is selected on a dish having a holding recess which can store a cell.

Specifically, a group of cells dispersed in a cell suspension is scattered on the dish by using a dispensation tip. The group of cells scattered here includes cells of various sizes and shapes. By capturing an image of the dish and executing image processing and the like, a cell suited for the processing work is selected from among these cells. The selected cell is picked from the dish by a tip capable of suctioning and discharging the cell and is also transferred to the microplate and discharged to the well. Before being scattered on the dish, the cell suspension is desirably subjected to filtering for removing cells of excessively large size and excessively small size which are apparently not appropriate for the treatment work. Patent Literature 1 mentions filtering of cancer cells.

Cells may have, for example, sensitivity, origin, and the like relative to an examination target greatly varying with their sizes. It is therefore desirable to conduct the treatment work on a similar size basis, and size selection is therefore often demanded at the time of the picking. In this case, when the cell suspension having been subjected to filtering to remove cells of excessively large size and excessively small size is scattered on the dish, the selection work requires labor. Specifically, although cells of excessively large size and excessively small size are removed, cells contained in the cell suspension vary so greatly in size that selection processing consumes time even if size selection is conducted by, for example, image processing.

### Citation List

### Patent Literature

Patent Literature 1: Japanese Patent No. 5652809

### Summary of Invention

An object of the present invention is to provide a pretreatment method which enables a required cell to be efficiently transferred by using a cell transfer device including cell picking means, and a cell transfer device to which the method is applied.

A pretreatment method for cell transfer according to one aspect of the present invention is a pretreatment method to be performed for transferring a required cell to a predetermined transfer destination by picking the cell, from a mass containing a plurality of cells of different sizes, using a cell transfer device including cell picking means, the pretreatment method including: a classification step of classifying the required cell, from among the plurality of cells contained in the mass, into at least two groups according to a size of the cell; and a supply step of supplying the classified cells, in a state of being individually classified, to a position where picking can be conducted by the picking means.

A cell transfer device according to another aspect of the present invention includes the cell picking means; moving means which causes the picking means to move to a predetermined direction; selection means which selects a cell to be a transfer target from among the cells supplied in the supply step; and an input unit which accepts input of a mesh size of the filter used, in which the selection means conducts the selection of a cell having a size according to the input mesh size based on a selection criterion determined in advance; the picking means picks a cell selected by the selection means; and the moving means causes the picking means having picked the cell to move to a predetermined movement destination.

### Brief Description of Drawings

FIG. 1 is a view schematically showing a cell transfer device according to an embodiment of the present invention.
FIG. 2 is a view showing cell dispensation operation to be executed before cell transfer work is conducted by the cell transfer device.
FIG. 3A is a top view of a dish provided in a selection container for use in the cell transfer device, and FIG. 3B is a sectional view taken along line IIIB-IIIB in FIG. 3A.
FIG. 4A is a perspective view of a microplate for use in the cell transfer device, and FIG. 4B is a sectional view of the microplate.
FIG. 5 is a block diagram showing an electrical configuration of the cell transfer device.
FIG. 6A is a side view of a cell filter, and FIG. 6B is a plan view of the cell filter viewed from the top.
FIG. 7 is a view for explaining cell filtering work using the cell filter.
FIG. 8 is a view showing a procedure of a pretreatment method for cell transfer according to the embodiment of the present invention.
FIG. 9 is a schematic view showing conventional cell picking work.
FIG. 10 is a schematic view showing cell picking work of the present embodiment.
FIG. 11 is a diagram showing a procedure of a pretreatment method for cell transfer according to a modification.
FIG. 12 is a view showing a preferred example of work from pretreatment for cell transfer until cell picking.
FIG. 13 is a view showing another preferred example of work from pretreatment for cell transfer until cell picking.

### Description of Embodiments

In the following, embodiments of the present invention will be described in detail with reference to the drawings. In a pretreatment method for cell transfer and a cell transfer device according to the present invention, a tissue-derived cell, a cell mass, a cellular aggregate (spheroid) or the like is used as a transfer target. For example, a tissue-derived cellular aggregate is formed by agglomerating several to several hundred thousands of cells. Therefore, a cellular aggregate varies in size. Although a cellular aggregate formed by a living cell is generally spherical, when a part of a cell constituting the cellular aggregate deteriorates or becomes a dead cell, the cellular aggregate may have a distorted shape or have uneven density in some cases. In a test for a biology-related technique or in a medical field, a cell transfer device is used which picks a usable cellular aggregate by a tip from among a plurality of cellular aggregates having various shapes which are carried by a dish on a selection stage, and transfers the picked cellular aggregate to a microplate. On the microplate, various processing is executed for the cellular aggregate, such as observation, checking of efficacy of medicines, examination, and culture. In the following description, in view of including such a cellular aggregate as described above, the cellular aggregate will be expressed simply as a cell C.

### [Overall Configuration of Cell Transfer Device]

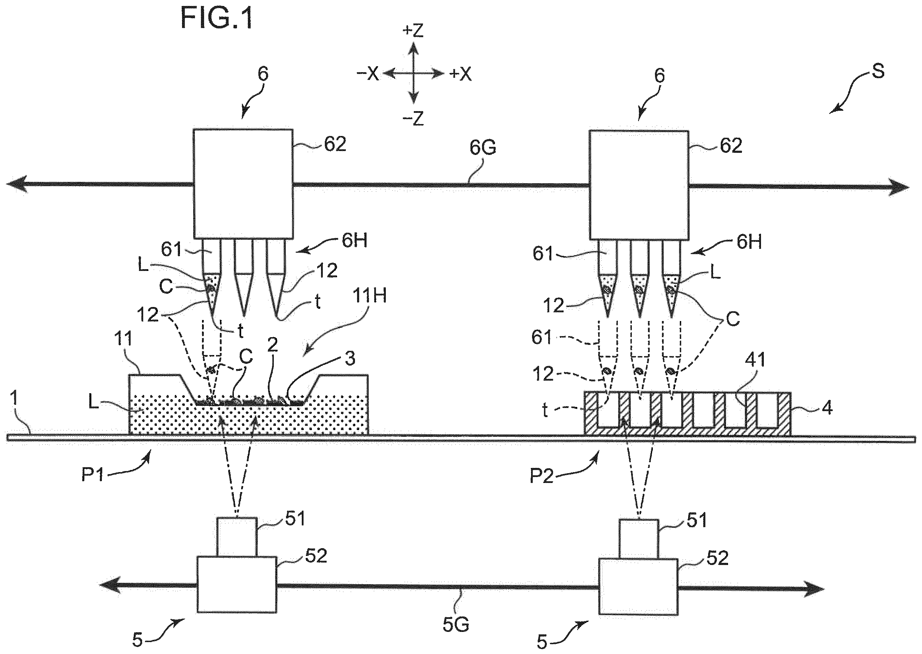

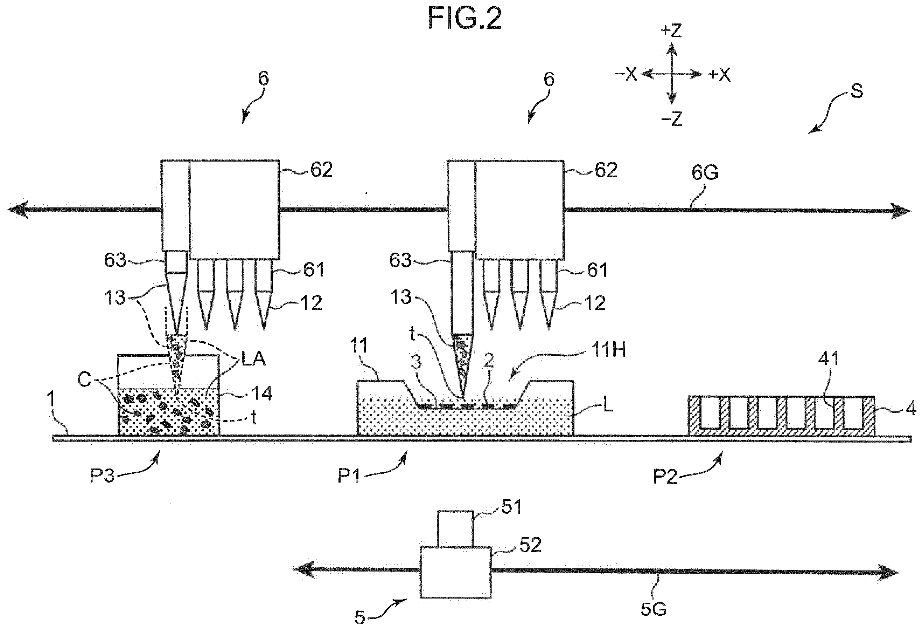

FIG. 1 and FIG. 2 are views schematically showing an overall configuration of a cell transfer device S. Here, the cell transfer device S which transfers a cell C among three containers is illustrated. The cell transfer device S includes a light transmissive base 1 having a level mounting surface (an upper surface), a camera unit 5 arranged below the base 1, and a head unit 6 arranged above the base 1. At a first mounting position P1 of the base 1, a selection container 11 provided with a dish 2 is mounted and at a second mounting position P2, a microplate 4 is mounted (FIG. 1). At a third mounting position P3 of the base 1, a dispensation container 14 is mounted (FIG. 2). The camera unit 5 and the head unit 6 are movable in an X direction and a Y direction (a direction orthogonal to the plane of the sheet of FIG. 1 and FIG. 2). The head unit 6 includes a plurality of heads 61 to which tips 12 (picking means) for suctioning and discharging a cell C for cell transfer is attached, and a dispensation head 63 to which a dispensation tip 13 for suctioning and discharging the cell C for cell dispensation is attached. The head 61 and the dispensation head 63 are movable in a Z direction.

Roughly explained, the cell transfer device S suctions a cell suspension LA containing numerous cells C from the dispensation container 14, scatters the cell suspension LA on the dish 2 of the selection container 11 (i.e., dispensation operation), selects a usable cell C on the dish 2, and suctions the usable cell C and also discharges the cell C to the microplate 4 (a well 41) (i.e., cell transfer operation). FIG. 1 shows an execution part of the cell transfer operation in the cell transfer device S. FIG. 2 is a view showing dispensation operation to be executed before cell transfer operation in FIG. 1 is conducted. For the dispensation operation, a pretreatment method for cell transfer according to the present invention is applied. In FIG. 1, illustration of the dispensation tip 13 is omitted for simplification of illustration. In the following, each part of the cell transfer device S will be described.

The base 1 is a rectangular flat plate having predetermined rigidity and a part or all of the base 1 is formed with a light transmissive material. The base 1 is preferably a glass plate. Forming the base 1 with a light transmissive material such as a glass plate allows the camera unit 5 arranged below the base 1 to capture the image of the selection container 11 (the dish 2) and the microplate 4 arranged on the upper surface of the base 1 through the base 1.

The dispensation container 14 is a container which has an upper surface opened and accumulates the cell suspension LA containing numerous cells C. The cell suspension LA includes cells C as a selection target in the selection container 11 and impurities which are inevitably mixed with cells C.

The selection container 11 is a container as a transfer source of the cell C, and accumulates a culture medium L and holds the cell selection dish 2 being immersed in the culture medium L. The dish 2 is a plate which holds the cell C and has, on its upper surface, a plurality of holding recesses 3 capable of individually storing and holding the cells C. The culture medium L is not particularly limited as long as it does not deteriorate properties of the cell C, and can be appropriately selected according to a kind of the cell C.

The selection container 11 is provided, on its upper surface side, with a rectangular upper opening 11H. The upper opening 11H is an opening for inserting the cell C and picking the selected cell C. The dish 2 is arranged below the upper opening 11H. The selection container 11 and the dish 2 for use are made of a light transmissive resin material or glass. This is for enabling the cell C carried on the dish 2 to be observed by the camera unit 5 arranged below the selection container 11.

The plurality of cells C being dispersed in the cell suspension LA are injected to the selection container 11 from the dispensation tip 13. At the third mounting position P3, the dispensation tip 13 suctions the cell suspension LA from the dispensation container 14 which accumulates the cell suspension LA containing a large number of cells C, and holds the suspension in the dispensation tip 13. Thereafter, the dispensation tip 13 is moved to a position above the selection container 11 (the first mounting position PI) to access the upper surface of the dish 2 through the upper opening 11H. Then, with a distal end opening portion t of the dispensation tip 13 immersed in the culture medium L of the selection container 11, the cells C dispersed in the cell suspension LA held in the dispensation tip 13 are discharged on the dish 2. Here, the cell suspension LA is subjected to filtering (pretreatment) for removing cells C of excessively large size and excessively small size which are apparently not appropriate for usages for examination or the like before being scattered on the dish 2. This will be described in detail later.

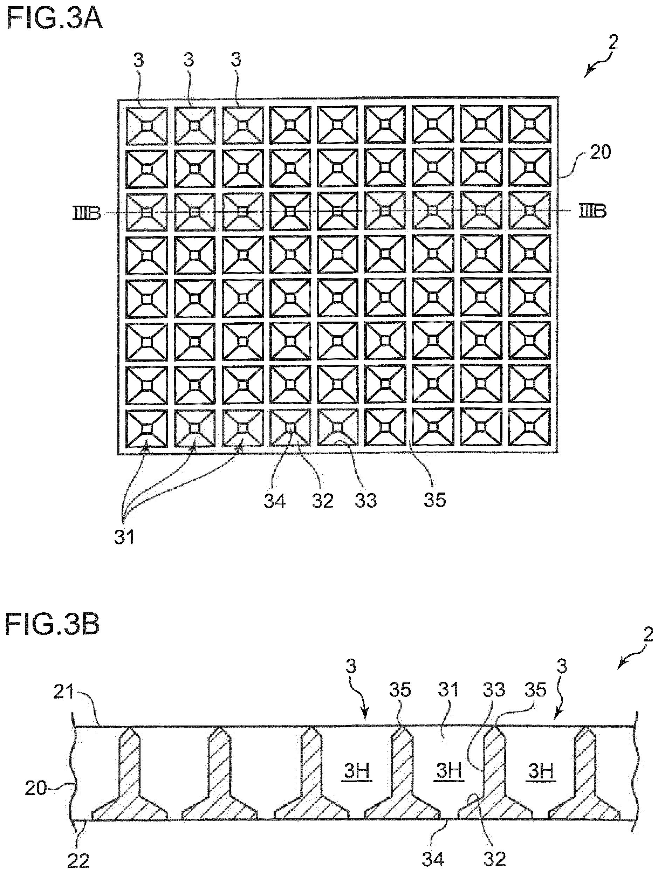

Detailed structure of the dish 2 will be described. FIG. 3A is a top view of the dish 2, and FIG. 3B is a sectional view taken along line IIIB-IIIB in FIG. 3A. The dish 2 is provided with a dish main body 20 and a plurality of holding recesses 3 formed in the dish main body 20. The dish main body 20 is made of a flat plate-shaped member having a predetermined thickness and has an upper surface 21 and a lower surface 22. The holding recess 3 has a reception opening (opening portion 31) for the cell C on the side of the upper surface 21. The dish 2 is immersed in the culture medium L in the selection container 11. Specifically, while the upper surface 21 of the dish main body 20 is immersed in the culture medium L in the selection container 11, the lower surface 22 is held in the selection container 11 in a state of being spaced from a bottom plate of the selection container 11 (see FIG. 1).

Each of the holding recesses 3 includes the opening portion 31, a bottom portion 32, a tubular wall surface 33, a hole portion 34 and a boundary portion 35. In the present embodiment, there is shown an example where the holding recesses 3 which are square in a top view are aligned in a matrix. The opening portion 31 is a square opening provided in the upper surface 21 and has a size which allows a distal end opening portion t of the tip 12 for selection to enter. The bottom portion 32 is positioned within the dish main body 20 and near the lower surface 22. The bottom portion 32 is an inclined surface gradually slanting toward the center (the center of the square). The tubular wall surface 33 is a wall surface extending vertically downward from the opening portion 31 toward the bottom portion 32. The hole portion 34 is a through-hole vertically penetrating between the center of the bottom portion 32 and the lower surface 22. The boundary portion 35 is a portion positioned in the upper surface 21 and corresponding to an opening edge of each holding recess 3 and is a ridgeline that partitions the holding recesses 3.

The bottom portion 32 and the tubular wall surface 33 of each holding recess 3 partition a storage space 3H which stores the cell C. The storage space 3H is in general intended to store one cell C. The hole portion 34 is provided for causing small cells and impurities of a size other than a desired size to escape from the storage space 3H. Accordingly, the hole portion 34 has a size selected to prevent the cell C of a desired size from passing through but allow small cells and impurities of a size other than the desired size to pass through. In this manner, the cell C to be selected is trapped in the holding recess 3, while impurities and the like drop from the hole portion 34 to the bottom plate of the selection container 11.

Returning to FIG. 1, the microplate 4 is a container which becomes a transfer destination of the cell C and has a plurality of wells 41 to which the cells C are discharged. The well 41 is a bottomed hole which is opened in an upper surface of the microplate 4. In one well 41, a necessary number (ordinarily one) of the cells C are stored together with the culture medium L. The microplate 4 used here is also made of a light transmissive resin material or glass. This is for enabling the cell C carried in the well 41 to be observed by the camera unit 5 arranged below the microplate 4.

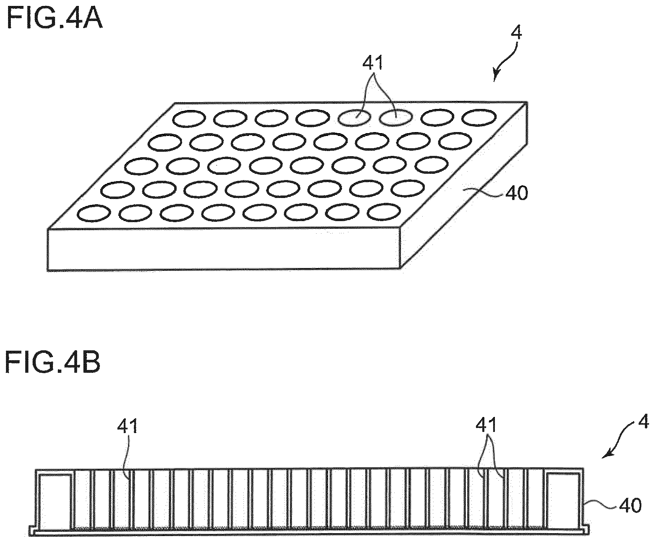

FIG. 4A is a perspective view showing one example of the microplate 4. The microplate 4 includes a plate main body 40 and the plurality of wells 41 aligned in the plate main body 40 in a matrix. Since the distal end opening portion t of the tip 12 enters the well 41 during discharge of the cell C, each well 41 has an opening diameter which allows the tip 12 to enter with a margin.

Commercially available microplates have a standard size. A standard microplate has a predetermined length-to-width size (length 85.48 mm × width 127.76 mm) and has a predetermined number of wells. A general number of wells is 24 × 16 (384 wells), the wells being aligned at a predetermined pitch in a matrix. FIG. 4B is a sectional view of the microplate 4 with 384 wells. As shown in the figure, 24 wells 41 are aligned at an equal well pitch in a longitudinal direction of the microplate 4 (16 in a shorter side direction).

The camera unit 5, which captures an image of the cell C held in the selection container 11 or the microplate 4 from their lower sides to obtain an image of the cell C, is provided with a lens unit 51 and a camera main body 52. The lens unit 51 is an objective lens for use in an optical microscope and includes a lens group which forms an optical image of a predetermined magnification, and a lens barrel which houses the lens group. The camera main body 52 is provided with an imaging element such as a CCD image sensor. The lens unit 51 forms an optical image of an imaging target on a light receiving surface of the imaging element.

The head unit 6 is provided for transferring the cell suspension LA from the dispensation container 14 to the selection container 11 and the cell C from the dish 2 to the microplate 4 and includes the plurality of heads 61 and one dispensation head 63, and a head main body 62 in which these heads are installed. The tip 12 (picking means) which conducts suctioning and discharging of the cell C is attached to a distal end of each head 61. The above dispensation tip 13 is attached to a distal end of the dispensation head 63. The head main body 62 holds the head 61 and the dispensation head 63 so as to be raised and lowered in +Z and -Z directions. Since the head unit 6 is movable in the XY directions as described above, the head unit 6 functions as moving means which causes the tip 12 and the dispensation tip 13 to move in a predetermined direction.

The head 61 and the dispensation head 63 are formed with a hollow rod having a negative pressure generation mechanism attached. In each of hollow portions of the head 61 and the dispensation head 63, for example, a piston mechanism is mounted, so that operation of the piston mechanism applies suction force and discharge force to the distal end opening portion t of the tip 12 or the distal end opening portion t of the dispensation tip 13. The head main body 62 is internally provided with a power unit of the piston mechanism, and a raising and lowering mechanism which causes the head 61 and the dispensation head 63 to move in the up-down direction and their power units (a head drive unit 65 to be described later).

### [Electrical Configuration of Cell Transfer Device]

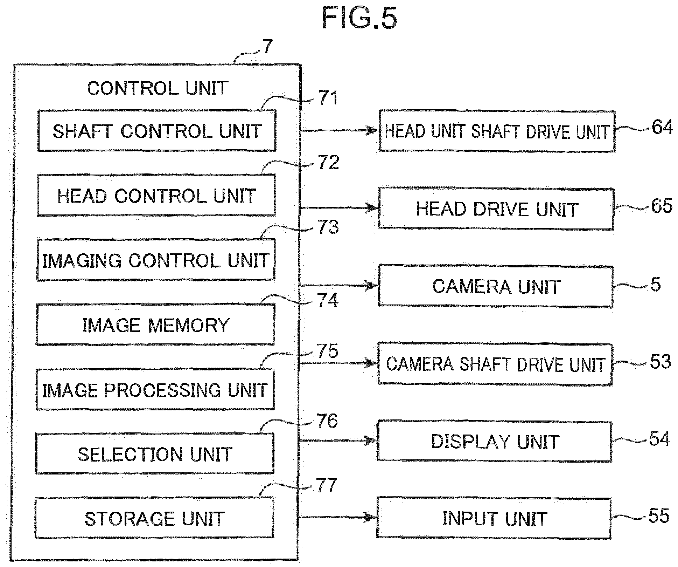

FIG. 5 is a block diagram showing an electrical configuration of the cell transfer device S. The cell transfer device S includes a control unit 7 which controls movement of the head unit 6, raising and lowering of the head 61 and the dispensation head 63, suctioning and discharging operations of the cell C, moving and imaging operations of the camera unit 5, and the like. The cell transfer device S also includes a camera shaft drive unit 53 as a mechanism which causes the camera unit 5 to move horizontally, a head unit shaft drive unit 64 (moving means) as a mechanism which causes the head unit 6 to move horizontally, and the head drive unit 65 as a mechanism which causes the head 61 and the dispensation head 63 to be raised and lowered and as a mechanism which causes the heads to conduct suctioning and discharging operations, and further includes a display unit 54 and an input unit 55.

The camera shaft drive unit 53 includes a drive motor which causes the camera unit 5 to move horizontally along a guide rail 5G. In a preferable mode, a ball thread is laid along the guide rail 5G, the camera unit 5 is attached to a nut member screwed with the ball thread, and the drive motor causes forward rotation or reverse rotation of the ball thread, thereby moving the camera unit 5 to a target position.

The head unit shaft drive unit 64 includes a drive motor which causes the head unit 6 (the head main body 62) to move along a guide rail 6G. In a preferable mode, with a ball thread and a nut member provided, the drive motor causes the ball thread to rotate forward or rotate reversely. In a case of moving the head main body 62 in the two directions of X and Y, a first ball thread (the X direction) along the guide rail 6G and a second ball thread (the Y direction) mounted on a moving plate attached to a first nut member screwed with the first ball thread are used. In this case, the head main body 62 is attached to a second nut member screwed with the second ball thread (the same applies to the camera shaft drive unit 53).

The head drive unit 65 is a power unit for the raising and lowering mechanism and a power unit (e.g. motor) for driving the piston mechanism and is incorporated in the head main body 62. The raising and lowering mechanism causes the head 61 or the dispensation head 63 to move up and down between a lowered position at which the head 61 or the dispensation head 63 protrudes downward from the head main body 62 and a raised position at which a most part of the heads is housed in the head main body 62. The power unit of the piston mechanism causes generation of suction force and discharge force at the distal end opening portion t of the tip 12 or the dispensation tip 13 by raising and lowering a piston member arranged in the head 61 or the dispensation head 63.

The display unit 54, which is configured by a liquid crystal display or the like, displays images such as an image captured by the camera unit 5 and an image subjected to image processing by the control unit 7.

An input unit 55, which is configured by a keyboard, a touch panel, or a communication unit which conducts data communication with other communication equipment, accepts input of operation information and various data from a user. In the present embodiment, the input unit 55 functions also as an input unit which accepts input of a mesh size of a filter used in a classification step of classifying a required cell C according to its size during the dispensation operation.

The control unit 7 is configured with a microcomputer or the like, and functions to include a shaft control unit 71 (a part of the moving means), a head control unit 72 (a part of the picking means), an imaging control unit 73, an image memory 74, an image processing unit 75 (a part of the selection means), a selection unit 76 (a part of the selection means), and a storage unit 77 as a result of execution of a predetermined program.

The shaft control unit 71 controls operation of the head unit shaft drive unit 64. Specifically, the shaft control unit 71 causes the head unit 6 to move to a predetermined target position in a horizontal direction by controlling the head unit shaft drive unit 64. Movement of the dispensation head 63 (the dispensation tip 13) between the dispensation container 14 and the selection container 11, movement of the head 61 (the tip 12) between the selection container 11 and the microplate 4, positioning of the head 61 vertically above the holding recess 3 of the dish 2, and positioning of the head 61 vertically above the well 41 of the microplate 4 as a discharge target, and the like are realized by the control of the head unit shaft drive unit 64 by the shaft control unit 71.

The head control unit 72 controls the head drive unit 65. The head control unit 72 causes the head 61 or the dispensation head 63 as a control target to be raised and lowered toward a predetermined target position by controlling the power unit for the raising and lowering mechanism of the head drive unit 65. The head control unit 72 also causes suction force or discharge force to be generated at the distal end opening portion t of the tip 12 or the dispensation tip 13 at predetermined timing by controlling the power unit of the piston mechanism for the head 61 or the dispensation head 63 as a control target.

The imaging control unit 73 controls the camera shaft drive unit 53 to control operation of moving the camera unit 5 along the guide rail 5G. The imaging control unit 73 also controls imaging operation of the dish 2 or the microplate 4 by the camera unit 5.

The image memory 74 is configured with a storage region provided in the microcomputer, an external storage, or the like and temporally stores image data obtained by the camera unit 5.

The image processing unit 75 subjects image data captured by the camera unit 5 and stored in the image memory 74 to image processing. By using an image processing technique, the image processing unit 75 executes processing of recognizing, on an image, presence of the cells C on the dish 2, processing of recognizing distribution of the cells C, processing of recognizing properties of the recognized cell C such as size, shape, color tone, and the like based on an image of the dish 2 to which the cells C have been dispensed.

The selection unit 76 selects a cell C to be a transfer target, i.e., selects a cell C to be transferred from the dish 2 to the microplate 4 based on a selection criterion determined in advance. The selection target is a cell C present on the dish 2, the cell C having its size, shape, color tone and the like specified by the image processing unit 75. It is desirable, during the above selection, to refer to a mesh size of a cell filter 8 to be described later, the mesh size having been accepted by the input unit 55.

The storage unit 77 stores various set values and data in the cell transfer device S. Other than these data, the storage unit 77 also stores data related to a selection criterion for each size of the cells C. Some cells C have different quality determination criteria varying with their sizes and it is desirable to provide a selection criterion obtained from, for example, a result of machine learning with respect to each size. For the above selection, the selection unit 76 reads the selection criteria stored in the storage unit 77 to conduct predetermined determination processing. The mesh size can be used as a trigger of the reading.

### [Operation of Cell Transfer Device]

Subsequently, a cell transfer method using the cell transfer device S according to the present embodiment will be described with reference to FIG. 1 and FIG. 2. First, a step of preparing necessary facility is executed. The selection container 11, the microplate 4, and the dispensation container 14 are mounted at the predetermined first to third mounting positions P1 to P3 within a movable range of the head unit 6. Our of a cell suspension containing cells cultured in a cell culture container or the like, a cell suspension LA subjected to pretreatment (to be described in detail later) of filtering a cell C of a required size is injected to the dispensation container 14.

First, dispensation operation of the cells C is executed. The shaft control unit 71 controls the head unit shaft drive unit 64 to cause the head unit 6 with the dispensation tip 13 attached to the dispensation head 63 to be moved to a position above the dispensation container 14 as shown in FIG. 2. Subsequently, the head control unit 72 controls the head drive unit 65 to cause the dispensation head 63 to be lowered, so that the distal end opening portion t of the dispensation tip 13 is immersed in the cell suspension LA of the dispensation container 14. In this state, the head drive unit 65 causes suction force to be generated at the dispensation head 63, resulting in suctioning the cell suspension LA into the dispensation tip 60.

Thereafter, the dispensation head 63 is raised, while the head unit 6 is moved to a position above the selection container 11. The dispensation head 63 is again lowered and the distal end opening portion t of the dispensation tip 13 accesses the upper surface 21 of the dish 2 through the upper opening 11H of the selection container 11. Then, with the distal end opening portion t being immersed in the culture medium L in the selection container 11, the cell suspension LA held in the dispensation tip 13 is discharged. In other words, the cells C are scattered on the dish 2.

Subsequently, selection operation of the cell C is executed. The imaging control unit 73 controls the camera shaft drive unit 53 to cause the camera unit 5 to move below the selection container 11 along the guide rail 5G. Then, the imaging control unit 73 controls the camera unit 5 to capture an image of the cell C carried on the dish 2 (FIG. 1). The obtained image data is temporally stored in the image memory 74 and is also subjected to predetermined image processing by the image processing unit 75. Thereafter, determination is made of selecting a cell C (good cell C) as a transfer target by the selection unit 76. The selected cell C is handled as a picking target by the tip 12 to obtain its coordinate position.

Subsequently, cell transfer operation is executed. The shaft control unit 71 controls the head unit shaft drive unit 64 to move the head unit 6 having the head 61 with the tip 12 attached thereto to a position above the selection container 11. Then, the head control unit 72 controls the head drive unit 65 to lower the head 61, so that the distal end opening portion t of the tip 12 accesses the upper surface of the dish 2 through the upper opening 1H. At this time, XYZ coordinate information indicative of a position of the cell C as a transfer target is given to the shaft control unit 71 and the head control unit 72, so that the tip 12 accesses the holding recess 3 where the cell C is carried.

Thereafter, the head drive unit 65 causes the head 61 to generate suction force. As a result, a target cell C is suctioned from the dish 2 (the holding recess 3) into the tip 12 together with the culture medium L (picking of the cell C). Then, the head 61 is raised and the head unit 6 is moved to a position above the microplate 4 (the picking means is moved to a predetermined movement destination).

When the head unit 6 arrives at the position above the microplate 4, the head 61 is again lowered until the distal end opening portion t of the tip 12 enters the well 41 of the microplate 4. Then, the head drive unit 65 causes the head 61 to generate discharge force, so that the cell C in the tip 12 is discharged to the well 41. A discharge status of these cells C is checked by imaging of the microplate 4 by the camera unit 5.

### [Pretreatment Method for Cell Transfer]

Subsequently, description will be made of a pretreatment method conducted when a required cell C is picked from the dish 2 by using such a cell transfer device S having the tip 12 as described above and is transferred to the microplate 4. The pretreatment here is processing of filtering, from a cell suspension LA as a mass containing a plurality of cells C and impurities of different sizes, a cell suspension LA containing only cells C of a required size and supplying the filtered cells to the dispensation container 14.

### <Cell Filter>

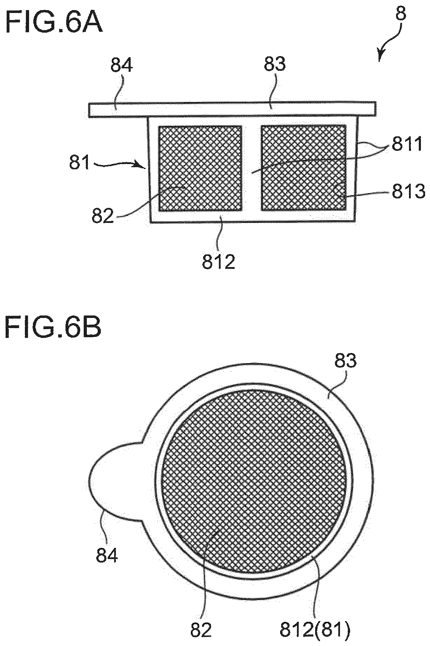

For the filtering, the cell filter 8 as shown in FIG. 6A and 6B is used, for example. FIG. 6A is a side view of the cell filter 8 and FIG. 6B is a plan view of the cell filter 8 viewed from the top. The cell filter 8 includes a cylindrical frame 81 and a filter film 82 adhered to the frame 81.

The frame 81 includes a plurality of side ribs 811 which are vertical ribs aligned in a circumferential direction, and a circular base rib 812 connecting lower ends of the side ribs 811. A flange portion 83 is attached to an upper end of the side rib 811. An opening portion 813 is present between the side ribs 811. An opening portion is also present inside the base rib 812. The filter film 82 is a film having meshes as minute openings and is adhered to the frame 81 so as to close the opening portion 813. A mesh size of the filter film 82 is selected according to a size of a cell C to be filtered. An operator handles the cell filter 8 by a grip portion 84 provided to protrude from the flange portion 83.

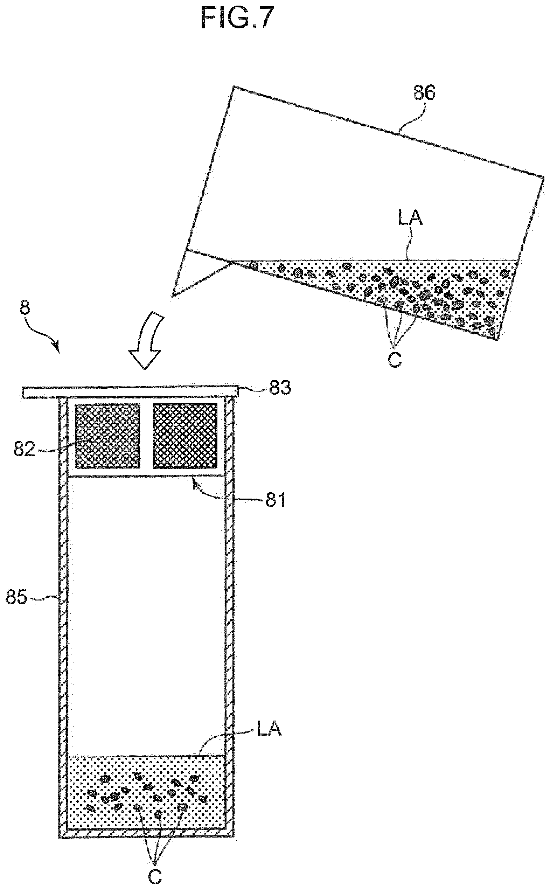

FIG. 7 is a view for explaining filtering work of a cell C using the cell filter 8. For filtering, there are prepared a tube 85 formed with a tubular container having an upper end opened, and a pot 86 which accumulates the cell suspension LA taken out from the cell culture container. The cell filter 8 is embedded into the tube 85 such that the flange portion 83 is supported by the upper end of the tube 85. Then, the cell suspension LA is poured from the pot 86 through an upper surface opening of the cell filter 8.

Then, the cell suspension LA containing cells C of a size which allows passage through meshes of the filter film 82 will be accumulated in the tube 85. In a case where the cell filter 8 is used for removing an unnecessary cell C, the cell suspension LA accumulated in the tube 85 will be supplied as it is to the dispensation container 14. By contrast, in a case where the cell filter 8 is used for trapping a necessary cell C, cells C which could not pass through the filter film 82 and were trapped by the cell filter 8 will be taken out, and dispersed in a cell culture solution, and the obtained cell culture solution will be supplied to the dispensation container 14.

### <Specific Example of Pretreatment>

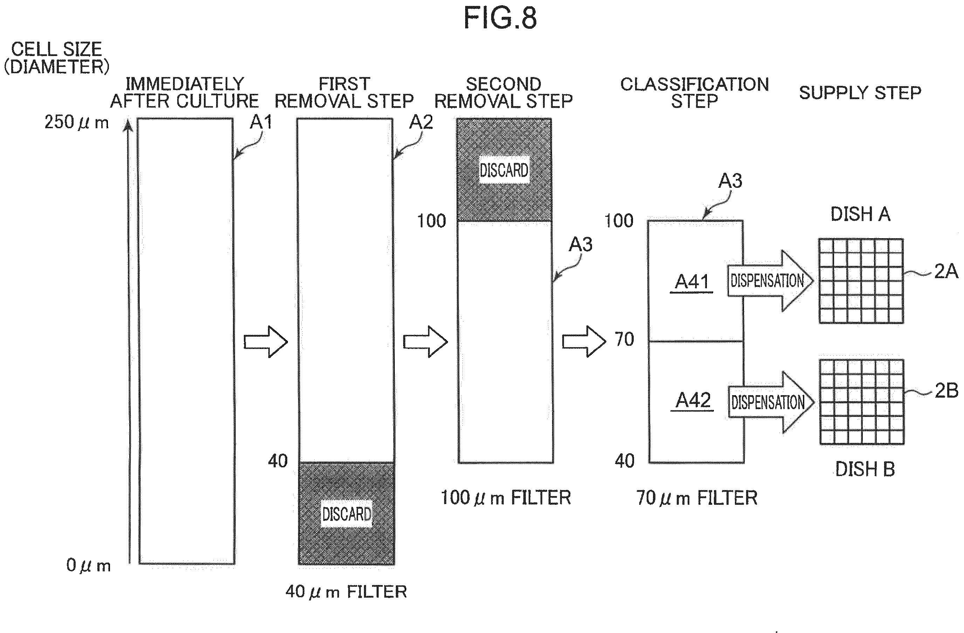

FIG. 8 is a view showing a procedure of the pretreatment method for cell transfer according to the embodiment of the present invention. The pretreatment method includes a first removal step, a second removal step, a classification step, and a supply step. In FIG. 8, a state of the cell suspension LA in each step is indicated by white boxes of the stages A1 to A4. A length of each box in a vertical direction represents variation in size (diameter) of a cell C contained in the cell suspension LA. FIG. 8 shows, for example, that the cell suspension LA in the stage A1 immediately after culture contains numerous cells C having a size within a range of above 0 µm and 250 µm or less.

The cell suspension LA in the stage A1 is a cell suspension (corresponding to "mass" in the present invention) in which cells C having a wide variation in size are dispersed and impurities and the like are also contained. For extracting a cell of a required size (required cell) from the cell suspension LA in the stage A1, the first removal step and the second removal step are executed. The required cell is a cell C having a size to be a target of examination or the like in the microplate 4, the cell C being of a size exceeding a predetermined minimum size (assumed to be 40 µm herein) and less than a predetermined maximum size (assumed to be 100 µm herein).

In the first removal step, filtering is executed for removing cells, impurities, and the like of a size equal to or less than the minimum size (40 µm) from the cell suspension LA in the stage A1. Accordingly, in the first removal step, the cell filter 8 with a mesh size of 40 µm is used. Specifically, the cell filter 8 with a mesh size of 40 µm is embedded in the tube 85 and a cell suspension LA in the stage A1 is stored in the pot 86.

Subsequently, the cell suspension LA is injected from the pot 86 into the tube 85 equipped with the cell filter 8 (40 µm). Then, a cell suspension LA in the stage A2 is obtained by taking out the cell trapped by the cell filter 8 and dispersing the taken out cell in the cell culture solution, or the like. The cell suspension LA filtered by the cell filter 8 and accumulated in the tube 85 is discarded.

In the second removal step, filtering is executed for removing cells C having a size equal to or more than the maximum size (100 µm) from the cell suspension LA in the stage A2. Accordingly, in the second removal step, the cell filter 8 with a mesh size of 100 µm is used. Specifically, the cell filter 8 with a mesh size of 100 µm is embedded in the tube 85 and a cell suspension LA in the stage A2 is stored in the pot 86.

Subsequently, the cell suspension LA is injected from the pot 86 into the tube 85 equipped with the cell filter 8 (100 µm). Then, the cell suspension LA having passed through the cell filter 8 and accumulated in the tube 85 is taken out to obtain a cell suspension LA in the stage A3. The cells C trapped by the cell filter 8 are discarded. The cell suspension LA in the stage A3 will be a cell suspension containing only required cells C as a target to be transferred to the microplate 4, i.e., cells C of a size of 40 µm to 100 µm in the present embodiment. The order of execution of the first and second removal steps is arbitrary and conversely to the example in FIG. 8, the second removal step can be executed prior to the first removal step.

In a conventional pretreatment method for cell transfer, pretreatment is finished at a stage where a cell suspension LA in the stage A3 is obtained. In other words, the cell suspension LA in the stage A3 is injected into the dispensation container 14. By contrast, in the present embodiment, a classification step is further executed with respect to the cell suspension LA in the stage A3. In the classification step, filtering is conducted for classifying required cells C contained in the cell suspension LA in the stage A3 into two groups according to their sizes. Here, an example is shown where cells C are classified into cells C of a size of 40 µm to 70 µm (a first size) and cells C of a size of 70 µm to 100 µm (a second size).

For the above classification, in the classification step, the cell filter 8 with a mesh size of 70 µm is used for allowing cells C of the first size to pass through, while not allowing cells C of the second size to pass through. Specifically, the cell filter 8 with a mesh size of 70 µm is embedded in the tube 85 and the cell suspension LA in the stage A3 is stored in the pot 86. Next, the cell suspension LA is injected from the pot 86 into the tube 85 equipped with the cell filter 8 (70 µm).

Then, the cells C trapped by the cell filter 8 are taken out and dispersed in the cell culture solution or the like to obtain a cell suspension LA in a stage A41 containing only cells C of a size of 70 µm to 100 µm. Also, the cell suspension LA having passed through the cell filter 8 and accumulated in the tube 85 is taken out to obtain a cell suspension LA in a stage A42 containing only the cells C of a size of 40 µm to 70 µm.

Thereafter, the supply step is executed for supplying the cells C classified into the cell suspension LA in the stage A41 and the cells C classified into the cell suspension LA in the stage A42 to a position at which picking by the tip 12 is possible, the cells C being supplied in a state of being respectively classified. The supply step in the present embodiment includes a step of injecting the cell suspensions LA in the stages A41 and A42 separately into separate dispensation containers 14 and a step of dispensing the cell suspensions LA into separate dishes 2A and 2B from these dispensation containers 14.

The dispensation containers 14 into which the cell suspensions LA in the stages A41 and A42 are injected are sequentially mounted at the third mounting position P3 of the base 1 or simultaneously aligned in parallel with each other. Then, after the cell suspensions LA in the stages A41 and A42 are separately suctioned from the respective dispensation containers 14 and transferred by the dispensation tip 13 to the position above the dishes 2A and 2B (the first mounting position PI), each cell suspension LA is dispensed. The dishes 2A and 2B may be independent dishes equipped with separate selection containers 11 or spaced regions on one dish 2 may be used as the dishes 2A and 2B.

### [Advantage of Execution of Classification Step]

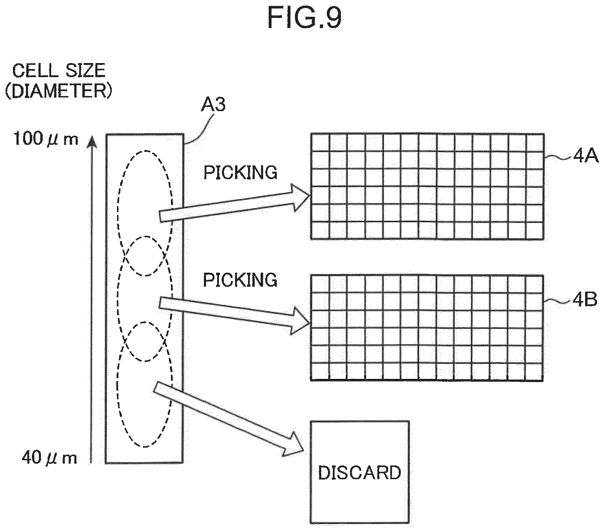

In the present embodiment, the above classification step is further executed with respect to the cell suspension LA in the stage A3. An advantage of execution of the classification step will be described. FIG. 9 is a schematic view showing cell picking work involving a pretreatment method of Comparative Example. In Comparative Example, the cell suspension LA in the stage A3 is dispensed to the dispensation container 14. Accordingly, the dish 2 has cells C of a size of 40 µm to 100 µm scattered and therefore, picking by the tip 12 brings about a need to select transfer target cells C from among those cells C.

For example, it is assumed that good cells C having a size of 80 µm ± 5 µm in the cell suspension LA in the stage A3 are picked from the dish 2 and transferred to a microplate 4A and good cells C having a size of 50 µm ± 5 µm are picked from the dish 2 and transferred to a microplate 4B (the remainder is discarded). In this case, in Comparative Example, processing will be required for selecting good cells C of a 80 µm class and good cells C of a 50 µm class from the dish 2 on which cells C in a wide range of sizes from 40 µm to 100 µm are scattered.

One of effective means of automatically determining quality of cells C is a machine learning method. In the machine learning, an algorithm is constructed which determines whether a cell is good or not, for example, by analyzing a large amount of sample data of images of cells C. However, a selection criterion for determining whether a cell is good or not may vary with a size of the cell C. For example, with respect to the above cell C of an 80 µm class and cell C of a 50 µm class, the selection criterion varies. Accordingly, when executing machine learning with respect to cells in a wide range of sizes, enormous sample data is necessary to cause a problem such as difficulty in constructing an algorithm or need of a large amount of processing time. According to the Comparative Example, since it is necessary to conduct sample data analysis with respect to cells C having the size of 40 µm to 100 µm as targets, and create an algorithm to be a selection criterion, introduction of machine learning involves difficulty.

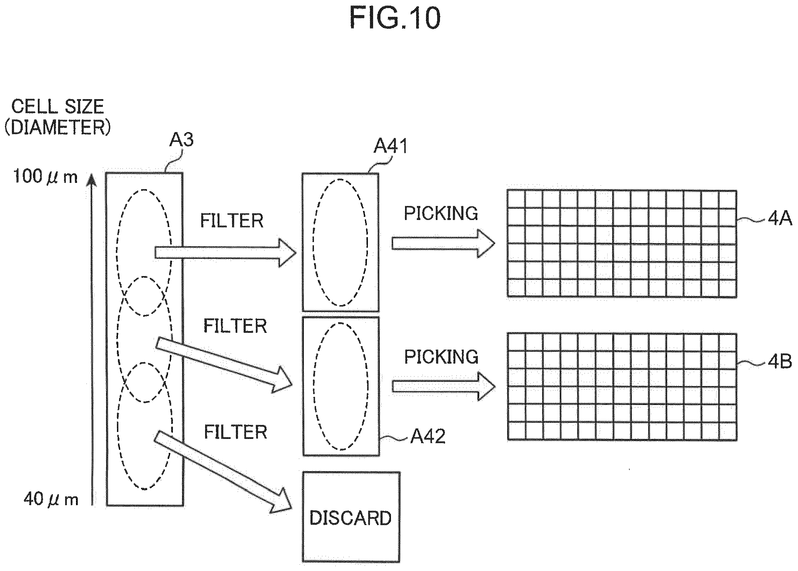

FIG. 10 is a schematic view showing cell picking work involving the pretreatment method of the present embodiment. In the present embodiment, the cell suspension LA in the stage A3 is classified into at least two groups according to a cell size in the classification step. Specifically, the cell suspension LA in the stage A3 is classified into two cell suspensions LA, i.e., the cell suspension LA in the stage A41 containing a group of cells at least having a large size and the cell suspension LA in the stage A42 containing a group of cells having a small size, and thus classified cell suspensions are supplied, in a state of being individually classified, to the microplates 4A and 4B, respectively, via the dispensation container 14.

Accordingly, the picking operation including the cell selection work by the cell transfer device S can be executed for a group of cells which have reduced variation of cell sizes. For example, in a case where the classification step (filtering) is executed for the cell suspension LA in the stage A3 by the cell filter 8 with a mesh size of 70 µm, selection work of the above-described cells C of an 80 µm class and cells C of a 50 µm class are considerably facilitated. Specifically, it is only necessary to select good cells C of a 80 µm class from the dish 2A to which the cell suspension LA in the stage A41 containing cells C of 70 µm to 100 µm is dispensed, and select good cells C of a 50 µm class from the dish 2B to which the cell suspension LA in the stage A42 containing cells C of 40 µm to 70 µm is dispensed. Accordingly, in a case of selecting cells by introducing a machine learning method, because of small variation in a cell size, an appropriate selection criterion can be obtained by a relatively small number of sample data to simplify processing.

### [Description of Other Embodiments]

### <Modification of Classification Step>

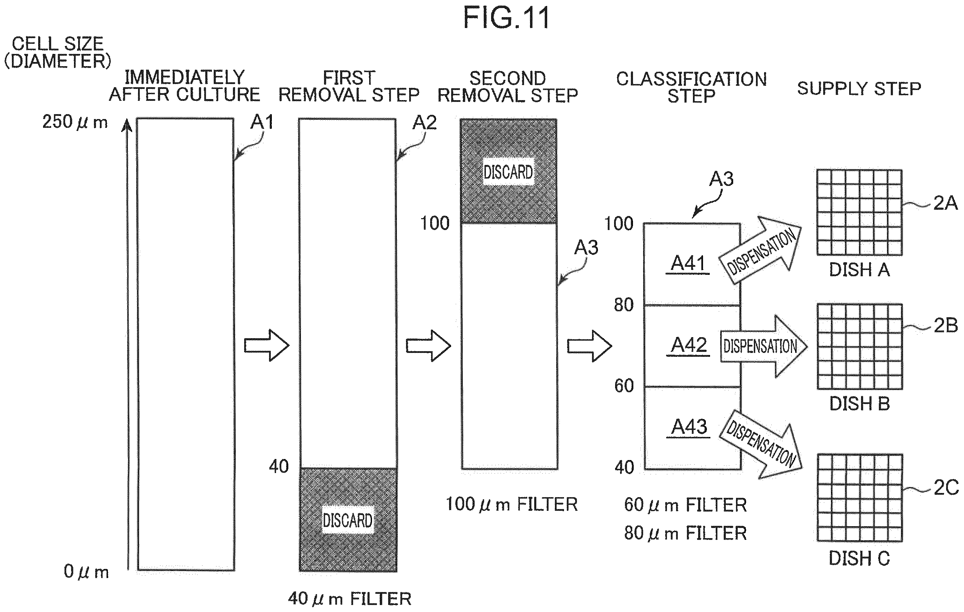

In FIG. 8, an example is shown in which the cell suspension LA in the stage A3 is classified into the two cell suspensions LA of the stages A41 and A42 in the classification step. In the classification step, the cell suspension LA in the stage A3 can be classified into three or more cell suspensions. FIG. 11 is a diagram showing a procedure of the pretreatment method for cell transfer according to a modification.

In the pretreatment method shown in FIG. 11, while the first and second removal steps are the same as the example shown in FIG. 8, in the classification step, filtering is executed for classifying required cells C contained in the cell suspension LA in the stage A3 into three groups according to a cell size. Here, an example is shown in which cells are classified into three groups, i.e., cells C of 80 µm to 100 µm, cells C of 60 µm to 80 µm, and cells C of 40 µm to 60 µm. In this case, two cell filters with a mesh size of 60 µm and 80 µm are used as the cell filter 8.

In one specific example of classification work, first, the cell filter 8 with a mesh size of 80 µm is embedded in the tube 85 and the cell suspension LA in the stage A3 is stored in the pot 86. Next, the cell suspension LA is injected into the tube 85 equipped with the cell filter 8 (80 µm) from the pot 86. Then, a cell suspension LA in the stage A41 containing only the cells C of 80 µm to 100 µm is obtained by taking out the cells C trapped by the cell filter 8 and dispersing the cells in the cell culture solution, and the like.

Then, the cell suspension LA having passed through the cell filter 8 and accumulated in the tube 85 is taken out to obtain a cell suspension LA containing only the cells C of 40 µm to 80 µm. Subsequently, the cell filter 8 with a mesh size of 60 µm is embedded in the tube 85 and the cell suspension LA containing the cells C of 40 µm to 80 µm is stored in the pot 86. The cell suspension LA is injected into the tube 85 equipped with the cell filter 8 (60 µm) from the pot 86.

Thereafter, a cell suspension LA in the stage A42 containing only the cells C of 60 µm to 80 µm is obtained by taking out the cells C trapped by the cell filter 8 and dispersing the cells in cell culture solution, and the like. The cell suspension LA having passed through the cell filter 8 and accumulated in the tube 85 is taken out to obtain a cell suspension LA in a stage A43 containing only the cells C of 40 µm to 60 µm. The obtained cell suspensions LA in the stages A41, A42, and A43 are dispensed to the different dishes 2A, 2B, and 2C.

### <Example of Use of Mesh Size in Control>

The cell transfer device S of the present embodiment includes the input unit 55 which accepts input of various data from a user (FIG. 5). The input unit 55 may be configured to accept input of a mesh size of the cell filter 8 used in the classification step such that the input mesh size is used as a selection criterion for a cell C in the selection unit 76.

With reference to the example in FIG. 8 above, the cell suspension LA in the stage A41 is a cell suspension LA obtained by two cell filters 8 with mesh sizes of 70 µm and 100 µm. In this case, the user inputs data of mesh sizes of 70 µm and 100 µm to the input unit 55. The selection unit 76 automatically sets a condition that a cell size (diameter) is 70 µm to 100 µm as one of selection criteria for a cell C. Further, the selection unit 76 reads other selection criterion (an algorithm by machine learning or the like) set related to a cell C with a size of 70 µm to 100 µm from the storage unit 77. Then, when the cell suspension LA in the stage A41 is dispensed to the dish 2A, the selection unit 76 makes quality determination of each cell C by applying the above selection criterion to each image of a cell C carried by the dish 2A whose image has been captured by the camera unit 5. This improves efficiency of the cell transfer work.

In addition to the above, the input unit 55 can further accept input of at least one of an upper limit and a lower limit of a size of a cell C as a transfer target such that the selection unit 76 handles the input upper limit or lower limit of the size of the cell C as a parameter of the selection criterion. Here, it is desirable that when the upper limit or the lower limit of the size input to the input unit 55 is inconsistent with the previously input mesh size of the cell filter 8, the selection unit 76 does not accept input of at least one of the upper limit and the lower limit of the size.

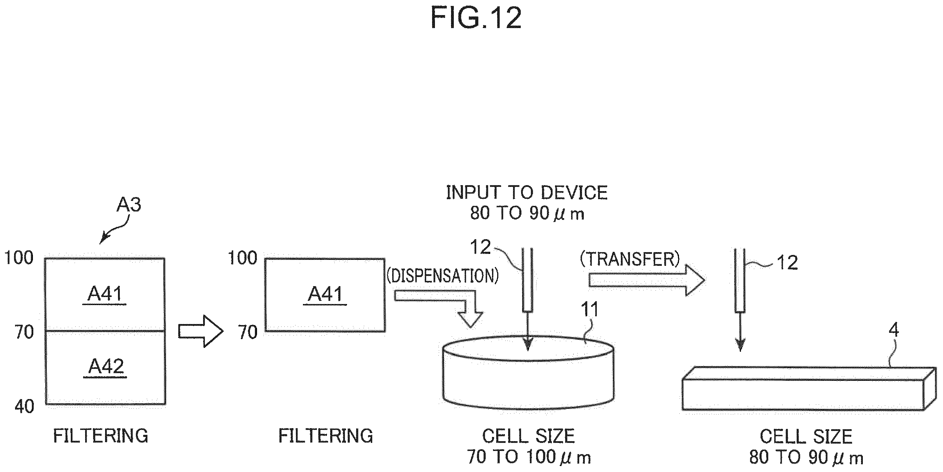

With reference to FIG. 12, a specific example will be described. FIG. 12 is a view showing a preferred example of work from the pretreatment for cell transfer until the cell picking. Similarly to the example shown in FIG. 8, it is assumed that a cell suspension LA in the stage A3 is obtained using the cell filters 8 with mesh sizes of 40 µm and 100 µm in the first and second removal steps, and cell suspensions LA in the stages A41 and A42 are obtained using the cell filter 8 with a mesh size of 70 µm in the classification step. Then, it is assumed that the cell suspension LA in the stage A41 is dispensed to the dish 2 of the selection container 11. Here, it is assumed that a cell C to be transferred to the microplate 4, i.e., a cell C to be a picking target has a size of 80 µm to 90 µm.

In this case, as described in the above example, the input unit 55 accepts input of data from the user, the data being that the cell filter 8 used for obtaining the cell suspension LA in the stage A41 has the mesh sizes of 70 µm and 100 µm. The input unit 55 further accepts input of data that a cell C to be a picking target has a size of 80 µm to 90 µm. Then, the selection unit 76 automatically sets a condition that the cell size is 80 µm to 90 µm as one of cell C selection criteria and reads other selection criterion set regarding the cell C having a size of 80 µm to 90 µm from the storage unit 77 to set a predetermined selection criteria.

Here, there might occur a case where with respect to a picking target cell size, the user inputs data that is inconsistent with the previously input mesh size (70 µm, 100 µm). That is a case, for example, where the user inputs the upper limit of 50 µm and the lower limit of 40 µm of a picking target cell size to the input unit 55. In this case, the selection unit 76 will not accept such input and urges the user to again input data by, for example, displaying an error message on the display unit 54.

When a mesh size and a picking target cell size are input to the input unit 55 without inconsistency, the selection unit 76 makes quality determination of a cell C based on a selection criterion according to the picking target cell size (80 µm to 90 µm). Then, the cell C selected as a transfer target is suctioned from the selection container 11 by the tip 12 and transferred to the microplate 4.

### <Modification of Dish and Microplate>

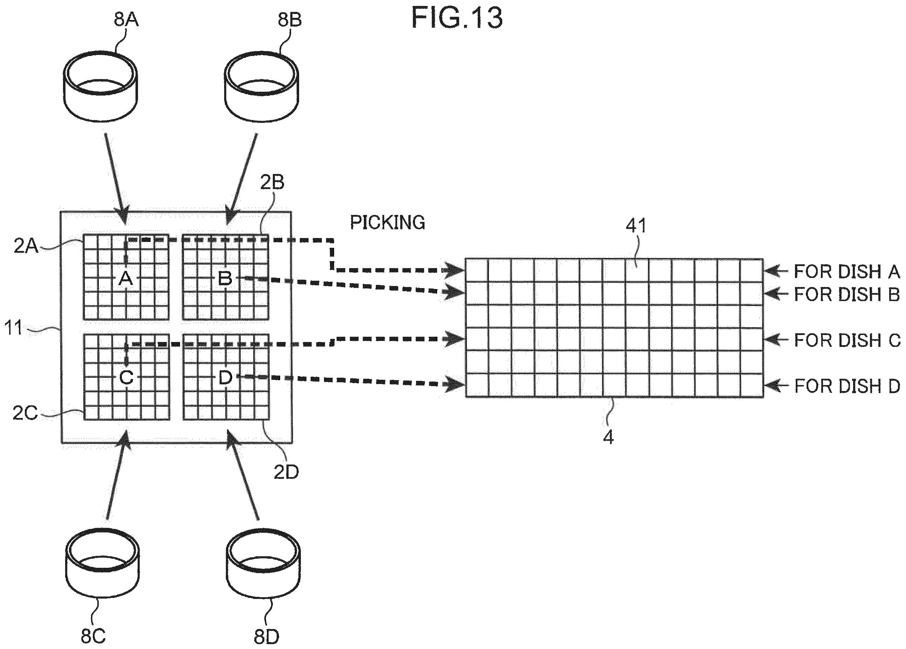

FIG. 13 is a view showing another preferred example of work from pretreatment for cell transfer until cell picking. Here, an example is shown where a plurality of dishes 2A to 2D to which cells C are dispensed are prepared, and in the microplate 4, the well 41 is assigned for each of the dishes 2A to 2D.

The four dishes 2A to 2D are attached to the selection container 11. While these dishes 2A to 2D are immersed in the culture medium L in the selection container 11 (see FIG. 1), the dishes are separated from each other by a partition wall or the like. In the dishes 2A to 2D, cells C of different sizes are dispensed. Specifically, the cell suspensions LA obtained by applying the cell filters 8A, 8B, 8C, and 8D with different mesh sizes are dispensed in respective sections in which the dishes 2A, 2B, 2C, and 2D are arranged.

The plurality of wells 41 provided in the microplate 4 are assigned in advance as a transfer destination of a cell C to be picked from each of the dishes 2A to 2D. Specifically, the assignment is made in advance such that a cell C picked from the dish 2A is assigned to the well 41 in a first row of the microplate 4 and a cell C picked from the dish 2B is assigned to the well 41 in a second row of the well 41.

Then, the cell C picked from each of the dishes 2A to 2D by the tip 12 is discharged to the well 41 among the plurality of wells 41, the well 41 being assigned to each of the dishes 2A to 2D from which the cell has been picked. This enables transfer of a required cell C to the well 41 in a state where a cell size is being classified or a state where a difference in a size of the cell C can be recognized. Accordingly, various kinds of subsequent treatment works on the microplate 4 can be appropriately executed according to a cell size.

The above-described specific embodiments mainly include the invention having the following components.

A pretreatment method for cell transfer according to one aspect of the present invention is a pretreatment method to be performed for transferring a required cell to a predetermined transfer destination by picking the cell, from a mass containing a plurality of cells of different sizes, using a cell transfer device including cell picking means, the pretreatment method including: a classification step of classifying the required cell, from among the plurality of cells contained in the mass, into at least two groups according to a size of the cell; and a supply step of supplying the classified cells, in a state of being individually classified, to a position where picking can be conducted by the picking means.

According to the pretreatment method, required cells are classified into at least two groups according to a cell size. As a result, the required cells are classified into at least two groups, a group of cells having a large size and a group of cells having a small size. Then, the required cells are supplied, in a state of being classified, to a position of the picking. Accordingly, picking operation including cell selection work by the cell transfer device at a subsequent stage can be executed for the groups of cells being classified. Specifically, since the picking operation can be executed with respect to a group of cells having reduced variation in cell size, working time can be reduced.

In the above pretreatment method, it is desirable that the required cell is a cell having a size exceeding a predetermined minimum size, and the pretreatment method further includes a first removal step of removing a cell having a size equal to or less than the minimum size from the mass.

It is also desirable that the required cell is a cell having a size less than a predetermined maximum size, and the pretreatment method further includes a second removal step of removing a cell having a size equal to or more than the maximum size from the mass.

According to these pretreatment methods, by the first and second removal steps, cells of excessively large size and excessively small size are removed from the mass. This enables only the required cell to be extracted from the mass. Accordingly, by the execution of the classification step, a plurality of cells within a range of a required size can be reliably classified further into at least two groups.

In the above pretreatment method, it is desirable that the mass is a cell suspension in which a plurality of cells of different sizes are dispersed, and in the classification step, a filter is used which allows a cell of a first size to pass, while not allowing a cell of a second size larger than the first size to pass.

According to the pretreatment method, classification work according to a size of the required cell in the classification step can be efficiently executed using the filter.

In the above pretreatment method, it is desirable that in the second removal step, a filter with a mesh size of 100 µm is used, and in the classification step, a filter with a mesh size of 70 µm is used.

There exist inexpensive general-purpose products having mesh sizes of 70 µm and 100 µm as a filter for filtering cells. Use of these filters enables removal of cells with a size of 100 µm or more in the second removal step from among the cells contained in the mass, and also enables classification of a group of cells with a size of less than 100 µm into two groups, i.e., a group of cells with a size of less than 70 µm and a group of cells with a size of 70 µm to 100 µm.

In the above pretreatment method, it is desirable that in the first removal step, a filter with a mesh size of 40 µm is used, in the second removal step, a filter with a mesh size of 100 µm is used, and in the classification step, a filter with a mesh size of 70 µm is used.

There also exists an inexpensive general-purpose product having a mesh size of 40 µm in addition to the above mesh sizes as a filter for filtering cells. Use of these filters enables removal of cells with a size of less than 40 µm in the first removal step and cells with a size of 100 µm or more in the second removal step, respectively, from among the cells contained in the mass. Further in the classification step, a group of cells with a size of 40 µm to 100 µm can be classified into two groups, i.e., a group of cells with a size of 40 µm to 70 µm and a group of cells with a size of 70 µm to 100 µm.

A cell transfer device according to another aspect of the present invention is a cell transfer device which is used after application of the above pretreatment method for cell transfer, the cell transfer device including: the cell picking means; moving means which causes the picking means to move to a predetermined direction; selection means which selects a cell to be a transfer target from among the cells supplied in the supply step; and an input unit which accepts input of a mesh size of the filter used, in which the selection means conducts the selection of a cell having a size according to the input mesh size based on a selection criterion determined in advance; the picking means picks a cell selected by the selection means; and the moving means causes the picking means having picked the cell to move to a predetermined movement destination.

According to the cell transfer device, the selection means conducts the selection based on a selection criterion determined in advance for a cell having a size according to the input mesh size. Here, since a cell size is made the same by the execution of the classification step, the selection criterion does not need to target cells in a wide range of size. For example, in a case where cells are selected by introducing a technique of machine learning, since cells have a small variation in size, an appropriate selection criterion can be obtained with a relatively small volume of sample data to simplify processing.

In the above cell transfer device, it is desirable that the input unit further accepts input of at least one of an upper limit and a lower limit of a size of a cell to be a transfer target, the selection means handles the upper limit or the lower limit of the size as a parameter of the selection criterion, and the input unit does not accept input of at least one of the upper limit and the lower limit of the size when the upper limit or the lower limit of the size is inconsistent with the mesh size.

According to the cell transfer device, when inconsistency is present, the input unit cannot accept input of an upper limit or a lower limit of the size. As a result, in addition to restriction of variation of a cell size by the input mesh size, variation of a cell size of cells as transfer targets will be further restricted.

In the above cell transfer device, it is desirable that in the supply step, a plurality of dishes capable of holding cells are prepared, each of the dishes holding cells on a classified cell basis, a microplate is arranged at the predetermined transfer destination, the microplate having a plurality of wells which accept cells, the picking means includes a tip which suctions and discharges the cell, and after a cell of each of the dishes is suctioned by the tip and transferred to the microplate, the cell is discharged from the tip to an assigned well among the plurality of wells.

According to the cell transfer device, it is possible to transfer the required cells to wells of the microplate in a state where sizes of the cells are being classified or where a difference in size of cells can be recognized. Accordingly, subsequent treatment work on the microplate can be appropriately executed according to a cell size.

The present invention described above provides a pretreatment method in which a required cell can be efficiently transferred by using a cell transfer device including cell picking means, and provides a cell transfer device to which such a method is applied.

## Claims

1. A pretreatment method for cell transfer to be performed for transferring a required cell to a predetermined transfer destination by picking the cell, from a mass containing a plurality of cells of different sizes, using a cell transfer device including cell picking means, the pretreatment method comprising:
a classification step of classifying the required cell, from among the plurality of cells contained in the mass, into at least two groups according to a size of the cell; and
a supply step of supplying the classified cells, in a state of being individually classified, to a position where picking can be conducted by the picking means.

2. The pretreatment method for cell transfer according to claim 1, wherein
the required cell is a cell having a size exceeding a predetermined minimum size, and
the pretreatment method further includes a first removal step of removing a cell having a size equal to or less than the minimum size from the mass.

3. The pretreatment method for cell transfer according to claim 1 or 2, wherein
the required cell is a cell having a size less than a predetermined maximum size, and
the pretreatment method further includes a second removal step of removing a cell having a size equal to or more than the maximum size from the mass.

4. The pretreatment method for cell transfer according to any one of claims 1 to 3, wherein
the mass is a cell suspension in which a plurality of cells of different sizes are dispersed, and
in the classification step, a filter is used which allows a cell of a first size to pass, while not allowing a cell of a second size larger than the first size to pass.

5. The pretreatment method for cell transfer according to 4 referring to claim 3, wherein
in the second removal step, a filter with a mesh size of 100 µm is used, and
in the classification step, a filter with a mesh size of 70 µm is used.

6. The pretreatment method for cell transfer according to 4 referring to claim 3, wherein
in the first removal step, a filter with a mesh size of 40 µm is used,
in the second removal step, a filter with a mesh size of 100 µm is used, and
in the classification step, a filter with a mesh size of 70 µm is used.

7. A cell transfer device which is used after application of the pretreatment method for cell transfer according to any one of claims 4, 5 and 6 which refer to claim 3, the cell transfer device comprising:
the cell picking means;
moving means which causes the picking means to move to a predetermined direction;
selection means which selects a cell to be a transfer target from among the cells supplied in the supply step; and
an input unit which accepts input of a mesh size of the filter used,
wherein the selection means conducts the selection of a cell having a size according to the input mesh size based on a selection criterion determined in advance;
the picking means picks a cell selected by the selection means; and
the moving means causes the picking means having picked the cell to move to a predetermined movement destination.

8. A cell transfer device according to claim 7, wherein
the input unit further accepts input of at least one of an upper limit and a lower limit of a size of a cell to be a transfer target,
the selection means handles the upper limit or the lower limit of the size as a parameter of the selection criterion, and
the input unit does not accept input of at least one of the upper limit and the lower limit of the size when the upper limit or the lower limit of the size is inconsistent with the mesh size.

9. The cell transfer device according to claim 7, wherein
in the supply step, a plurality of dishes capable of holding cells are prepared, each of the dishes holding cells on a classified cell basis,
a microplate is arranged at the predetermined transfer destination, the microplate having a plurality of wells which accept cells,
the picking means includes a tip which suctions and discharges the cell, and
after a cell of each of the dishes is suctioned by the tip and transferred to the microplate, the cell is discharged from the tip to an assigned well among the plurality of wells.
